# EUROPEAN PATENT APPLICATION

(11) **EP 2 592 103 A1**
(43) Date of publication of application: **15.05.2013**
(21) Application number: 11008884.6
(22) Date of filing: 08.11.2011
(51) Int. Cl.: C08G 65/329, C08G 65/333, A61K 47/48, C08G 65/331

(54) **Polymer aldehyde derivatives**

(71) Applicant: Adriacell S.p.A., 34149 Trieste (IT)
(72) Inventor: Kuehne, Christian, 1050 Wien (AT); Pasut, Gianfranco, 35132 Padova (IT)
(74) Representative: Forstmeyer, Dietmar

(57) **Abstract**

The present invention concerns polymer aldehyde derivatives, methods for producing them and their use for producing conjugates for diagnostic and/or therapeutic applications.

## Description

### Field of the invention

The present invention concerns polymer aldehyde derivatives, methods for producing them and their use for producing conjugates for diagnostic and/or therapeutic applications.

### Background of the invention

Polymer therapeutics encompass polymer-protein conjugates, drug-polymer conjugates, and supramolecular drug-delivery systems. These polymer therapeutics can accomplish several desirable objectives: a longer in vivo half-life; reduced immunogenicity, toxicity, and clearance rate through the kidneys; successful transportation across a cell membrane; protection against proteolysis; modification of electro-osmotic flow; increased pH and thermal stability; a low volume of distribution and sustained adsorption from the injection site; and improved formulation properties of the protein. These superior properties can increase effective potency, improve response to the drug, increase patient tolerance and reduce side effects, and reduce overall dosage. In recent years, numerous polymer therapeutics have been developed and some of them have already received market approval. An overview of polymer therapeutics is, for example, presented in Polymer Therapeutics I and II: Polymers as Drugs, Conjugates and Gene Delivery Systems (Advances in Polymer Science; eds. R. Satchi-Fainaro and R. Duncan; Springer Berlin Heidelberg; 2006).

Many polymer therapeutics also comprise one or more linker(s) having two or more reactive sites for binding (a) small molecule drug(s) (i.e. drugs having a molcular weight of less than 1000 Da) to the polymer. For example, antibody-drug-conjugates (also called immunoconjugates) are constituted by a recombinant antibody covalently bound by a synthetic linker to a given cytotoxic agent. Various classes of linkers have been developed and are known in the art, including linear and branched peptide linkers, thioether linkers, disulfide-based linkers, and acid-labile hydrazone linkers.

One of the most commonly employed polymers in polymer therapeutics is polyethylene glycol (hereinafter also referred to as PEG). PEG is approved for human administration by various routes of administration, e.g. mouth, injection, or dermal application. The structure of PEG is HO- (CH₂-CH₂-O)ₙ-H, where n indicates the number of repeats of the ethylene oxide unit in the PEG. PEG is a linear or branched, neutral polyether, and is commercially available in a variety of molecular weights; the polymerization can be controlled such that the molecular weight distribution is narrow.

Many of the benefits of PEGylated therapeutics lie in the properties of PEGs. PEGs are neutral, hydrophilic polymers that are soluble in water and a variety of organic solvents. Further, PEGs are inert, non-toxic, non-immunogenic, and the polymer is easily cleared from the body, mainly through the kidney for molecules with a molecular weight below 20 kDa, or through a combination of kidney and liver for molecules with molecular weight above 20 kDa. Up to day, the maximum PEG molecular weight used for the preparation of polymer therapeutics is 40 kDa.

A variety of PEG derivatives has been developed for such applications. Such PEG derivatives are described, for example, in US 5,252,714; US 5,990, 237; WO 00/24697; and WO 2004/013205.

Overall, covalent conjugation of polymers, e.g. PEG, with small molecule drugs and/or proteins is a promising approach for pharmaceutical applications, since such conjugates display altered (improved) pharmacokinetic properties, including a longer in vivo half-life; reduced immunogenicity; reduced toxicity; protection against proteolysis; improved water solubility; and increased pH and thermal stability; while the biological activity of the small molecule drug and/or the protein is commonly retained in those conjugates.

It would, therefore, be advantageous to provide novel, alternative polymer aldehyde derivatives that are on one hand suitable for conjugation with biomolecules containing an α-amino group, e.g. polypeptides of natural or synthetic origin including antibodies and other proteins, and, on the other hand, can be linked to small molecule drugs, e.g. cytotoxic drugs. There also remains a need to provide polymer aldehyde derivatives that can be easily produced in high yield and purity, and that can be selectively conjugated to various molecules, including biomolecules containing an α-amino group and small molecule drugs.

### Description of the invention

The present invention provides a variety of compounds and chemical intermediates which may be used in connection with the conjugation of hydrophilic macromolecular compounds of the invention to various materials and molecules, including polypeptides and other biomolecules.

In particular, the present invention relates to a compound of the general formula (I): 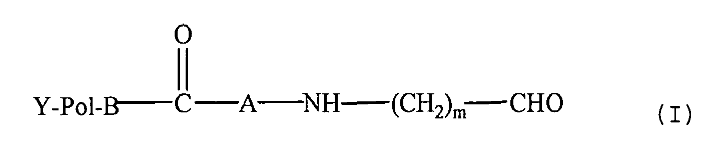 wherein:
Y is a capping group;
Pol is a water-soluble polymer of synthetic or natural origin;
B is N or O;
m is an integer of 1 to 4;
A is a spacer selected from:
-NH- (CH₂)ₚ-CO-; 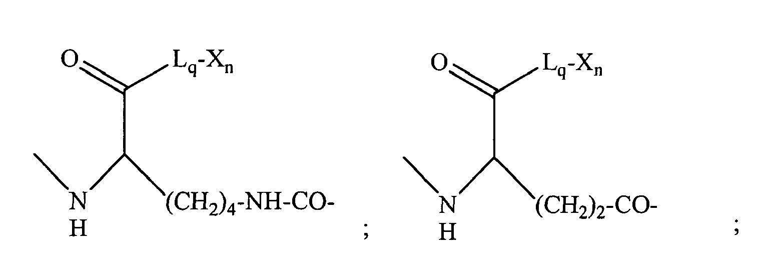 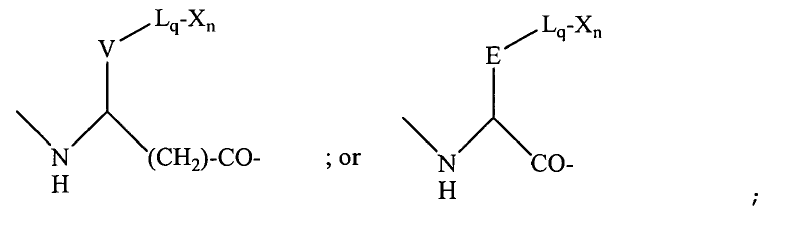
E is the side chain of lysine, cysteine, homocysteine, serine, threonine, ornithine, 2,4 diaminobutyric acid, glutamic acid, aspartic acid, or adipic acid;
V is the side chain of beta-lysine, beta-homolysine or beta-glutamic acid;
L, if present, represents a linker;
X, if present, represents a small molecule drug having a molecular weight of 300 to 1000 Da, a targeting residue, a protecting group, an activated group, or a labeling group, which is bound covalently, or via the respective linker L, to the carbonyl moiety (CO), or the side chain functional group (SH, COOH, NH₂) of E or V, wherein the covalent bond is formed via an ester, amide, carbamate, urea, ether, thioether, sulfur or disulfide bond;
n represents an integer of 0 to 10;
p is an integer of 2 to 10;
q represents an integer of 0 or 1.

The term "capping group" as used herein means any suitable chemical group which, depending upon preference, is unreactive or reactive with other chemical moieties. Accordingly, the capping group is selected to provide monofunctionality, i.e. the terminal aldehyde group, or bifunctionality, i.e. an aldehyde group on one terminus and a different functional moiety on the opposite terminus. If the capping group is unreactive with other chemical moieties, then the structure of the resulting polymer aldehyde derivative is monofunctional and therefore can covalently bond with only one chemical moiety of interest. In other words, in the case that the capping group is unreactive, the terminal aldehyde group of the compound of the present invention permits ready covalent attachment to a chemical moiety of interest, for example, to the α-amino group of a polypeptide. Suitable capping groups are generally known in the art, for example, those disclosed in WO 2004/013205. Suitable unreactive capping groups include, for example, alkoxy, e.g. methoxy, ethoxy, propoxy, or butoxy; halogen atom; or tosylate; and suitable reactive capping groups include, for example, isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or aldehydes.

The term "halogen atom" as used herein refers to fluorine, chlorine, bromine, and iodine atom.

The terms "group," "functional group," "moiety," "active moiety," "reactive site," and "radical" are somewhat synonymous in the chemical arts and are used in the art and herein to refer to distinct, definable portions or units of a molecule and to units that perform some function or activity and are reactive with other molecules or portions of molecules. In this sense a small molecule drug or a protein residue can be considered a functional group or moiety when coupled to a compound of the present invention.

The term "small molecule drug" as used herein preferably refers to a medicinal organic compound having a molecular weight of less than 1000 daltons, typically of 300 to 1000 daltons, and preferably of 300 to 700 daltons. The small molecule drug activates or inhibits the function of a biomolecule which in turn results in a therapeutic benefit to a patient, e.g. a mammal, preferably a human. This is, the small molecule drug usually binds with high affinity to a biomolecule such as a protein, nucleic acid, or polysaccharide and alters the activity or function of the biomolecule. The small molecule drug can be natural (such as secondary metabolites, including alkaloids, terpenoids, steroids, glycosides, natural phenols, phenazines, polyketides, fatty acid synthase products, nonribosomal peptides, macrolactones, and polyphenols) or artificial.

The preferred small molecule drugs for use in the present invention are cytotoxic agents, particularly those which are used for cancer therapy. Such drugs include, in general, DNA damaging agents, anti-metabolites, natural products and their analogs.

Preferred classes of cytotoxic agents include, for example, the enzyme inhibitors such as natural or synthetic tubulysins; natural or synthetic epothilones; duocarmycines; auristatins; maytansinoids; calicheamycin; mesothelins; anthracyclins; dihydrofolate reductase inhibitors; and thymidylate synthase inhibitors; DNA intercalators; DNA cleavers; topoisomerase inhibitors; the vinca drugs; the mitomycins; the bleomycins; the cytotoxic nucleosides; the pteridine family of drugs; diynenes; the podophyllotoxins; differentiation inducers; and taxanes. Useful members of those classes include, for example, erlotinib (TARCEVAR^{®}, Genentech/OSI Pharm.), docetaxel (TAXOTERE^{®}, Sanofi-Aventis), 5-FU (fluorouracil, 5-fluorouracil, CAS No. 51-21-8), gemcitabine (GEMZAR^{®}), Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL^{®}, Bristol-Myers Squibb Oncology, Princeton, N.J.), trastuzumab (HERCEPTIN^{®}, Genentech), temozolomide (4-methyl-5-oxo-2,3,4,6,8-pentazabicyclo [4.3.0] nona-2,7,9-triene-9-carboxamide, CAS No. 85622-93-1, TEMODAR^{®}, TEMODAL^{®}, Schering Plough), tamoxifen ((Z)-2-[4-(1,2-diphenylbut-1-enyl)phenoxy]-N,N-dimethyl-ethanamine, NOLVADEX^{®}, ISTUBAL^{®}, VALODEX^{®}), and doxorubicin (ADRIAMYCIN^{®}), Akti-1/2, HPPD, and rapamycin. Also useful cytotoxic agents include: oxaliplatin (ELOXATIN^{®}, Sanofi), bortezomib (VELCADE^{®}, Millennium Pharm.), sutent (SUNITINIB^{®}, SU1 1248, Pfizer), letrozole (FEMARA^{®}, Novartis), imatinib mesylate (GLEEVEC^{®}, Novartis), XL-518 (Mek inhibitor, Exelixis, WO 2007/044515), ARRY-886 (Mek inhibitor, AZD6244, Array BioPharma, Astra Zeneca), SF-1 126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX^{®}, AstraZeneca), leucovorin (folinic acid), rapamycin (sirolimus, RAPAMUNE^{®}, Wyeth), lapatinib (TYKERB^{®}, GSK572016, Glaxo Smith Kline), lonafarnib (SARASAR™, SCH 66336, Schering Plough), sorafenib (NEXAVAR^{®}, BAY43-9006, Bayer Labs), gefitinib (IRESSA^{®}, AstraZeneca), irinotecan (CAMPTOSAR^{®}, CPT-11, Pfizer), tipifarnib (ZARNESTRA™, Johnson & Johnson), ABRAXANE™ (Cremophor-free), vandetanib (rINN, ZD6474, ZACTIMA^{®}, AstraZeneca), chlorambucil, AG1478, AG1571 (SU 5271; Sugen), temsirolimus (TORISEL^{®}, Wyeth), pazopanib (GlaxoSmithKline), canfosfamide (TELCYTA^{®}, Telik), thiotepa and cyclosphosphamide (CYTOXAN^{®}, NEOSAR^{®}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, calicheamicin gamma 1, calicheamicin omega 11 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE^{®}); novantrone; teniposide; edatrexate; daunomycin; aminopterin; capecitabine (XELODA^{®}, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above. Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, 4-hydroxytamoxifen, trioxifene, keoxifene, LY117018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RIVISOR^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE^{®}, Genta Inc.); (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUKIN^{®} rIL-2; topoisomerase 1 inhibitors such as LURTOTECAN^{®}; ABARELIX^{®} rmRH; (ix) anti-angiogenic agents such as bevacizumab (AVASTIN^{®}, Genentech); leurosine, carminomycin, tallysomycin, podophyllotoxin, retinoic acid, butyric acid, N⁸-acetyl spermidine, and pharmaceutically acceptable salts, acids and analogues of any of the above.

The term "linker" or "link" as used herein means a divalent chemical moiety comprising a covalent bond or a chain of atoms that covalently attaches a small molecule drug moiety to the polymer aldehyde derivative of the present invention. The drug moiety has at least one chemically reactive functional group (e.g. a primary amine or secondary amine, hydroxyl, sulfhydryl, carboxyl, aldehyde or ketone) pendant thereto chemically reacted to the linker to form a covalent bond. In the compounds of the present invention as represented by general formula I, a linker is specified as L. Suitable linkers that can be emploeyed in the present invention are generally known in the art and include, for example, those described in EP 0328147; EP 0457250; EP 1156059; EP 1414792; EP 1434778; EP 1718667; EP 1771467; EP 1816192; EP 1912671; EP 2079840; EP 2118127; EP 2090323; EP 2265283; EP 2266986; US 6,759,509; US 2010/0034837; WO 02/051862; WO 2005/005464; WO 2010/128141; Doronina et al (2006) Bioconj. Chem. 17:114-124; Erickson et al (2010) Bioconj. Chem. 21:84-92; and Ducry and Stump (2010) Bioconj. Chem. 9: 665-667.

The term "targeting residue" as used herein means a moiety that delivers the polymer aldehyde derivative or conjugates formed therewith to a specific moiety of interest, e.g. specific organelles or cellular structures, including tumor tissue or tumor cells. Suitable targeting residues include, for example, hormones, folic acid, vitamins, alendronate, biphosphonates, galactosamine, antibodies and antibody fragments.

The term "protecting group" as used herein has the same meaning as commonly understood in the art and is consistent with: Greene's Protective Groups in Organic Synthesis, 4th ed., Peter G. M. Wuts and Theodora W. Greene, John Wiley & Sons (2006; ISBN: 978-0-471-69754-1). Suitable protecting groups include, for example, BOC, FMOC, and trityl.

The term "activated group" as used herein has the same meaning as commonly understood in the art, and includes, for example, N-hydroxysuccinimide activated carboxylic group, imidazole activated carboxylic group, ortho pirydine disulfide, maleimide, iodoacetaamide, bromoacetamide, hydrazine, aldehyde, azide, alkyne, and amine group.

The term "labeling group" as used herein refers to a label, or tag, or tracer, or marker, i.e. an easily recognizable chemical moiety, which allows to follow the translocation and/or the chemical or biological transformation of the moiety of interest, e.g. of a conjugate formed with the compound of the present invention. Suitable labeling groups include, for example, affinity lables, including antibodies and antibody fragments, radioactive labels, and fluorophores. Particularly usefule examples of a labeling group include: fluorescein, Cy5.5 and related cyanines emitting in the near UV.

The term "biomolecule" as used herein refers to any molecule that is produced by a living organism, including large polymeric molecules such as proteins, including antibodies, enzymes, proteins involved in the process of cell signaling and signal transduction, and membrane proteins that act as receptors, polysaccharides, lipids, and nucleic acids as well as primary metabolites, secondary metabolites, and natural products.

The terms "protein", "polypeptide", "peptide" as used herein define an organic compound made of two or more amino acid residues arranged in a linear chain, wherein the individual amino acids in the organic compound are linked by peptide bonds, i.e. an amide bond formed between adjacent amino acid residues. By convention, the primary structure of a protein is reported starting from the amino-terminal (N) end to the carboxyl-terminal (C) end.

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g., IgG, IgE, IgM, IgD, and IgA), class (e.g., IgG1, IgG2, IgG3, IgG4, IgA1 and IgA2) or subclass of immunoglobulin molecule. The immunoglobulins can be derived from any species, including human, murine, or rabbit origin.

"Antibody fragments" comprise a portion of a full length antibody, generally the antigen binding or variable region thereof. Examples of antibody fragments include Fab, Fab', F(ab')₂, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al (1975) Nature 256:495, or may be made by recombinant DNA methods (see, U.S. Pat. No. 4,816,567). The monoclonal antibodies may also be isolated from phage antibody libraries using the techniques described in Clackson et al (1991) Nature, 352:624-628; Marks et al (1991) J. Mol. Biol., 222:581-597. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; and Morrison et al (1984) Proc. Natl. Acad. Sci. USA, 81:6851-6855). Chimeric antibodies include "primatized" antibodies comprising variable domain antigen-binding sequences derived from a non-human primate (e.g., Old World Monkey or Ape) and human constant region sequences.

An "intact antibody" herein is one comprising a VL and VH domains, as well as a light chain constant domain (CL) and heavy chain constant domains, CH1, CH2 and CH3. The constant domains may be native sequence constant domains (e.g., human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called α, β, ε, γ, and µ respectively. The subunit structures and three-dimensional configurations of different classes of immunoglobulins are well known.

Particularly useful antibodies are therapeutic antibodies such as alemtuzumab (Campath), bevacizumab (AVASTIN^{®}, Genentech); cetuximab (ERBITUX^{®}, Imclone); panitumumab (VECTIBIX^{®}, Amgen), rituximab (RITUXAN^{®}, Genentech/Biogen Idec), pertuzumab (OMNITARG™, 2C4, Genentech), trastuzumab (HERCEPTIN^{®}, Genentech), tositumomab (Bexxar, Corixia), and the humanized monoclonal antibodies alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

The term "amino acid" as used herein refers to any organic acid containing one or more amino substituents, e.g. α-, β- or γ-amino, derivatives of aliphatic carboxylic acids. Preferably, the term "amino acid" refers to the 22 most common, natural L-amino acids, which are selected from the group consisting of glycine, leucine, isoleucine, valine, alanine, phenylalanine, tyrosine, tryptophan, aspartic acid, asparagine, glutamic acid, glutamine, cysteine, methionine, arginine, lysine, proline, serine, threonine, histidine, selenocysteine, and pyrrolysine. Of these, the 20 amino acids encoded by the universal genetic code can herein also be referred to by their conventional three- letter or one-letter abbreviations and their abbreviations follow conventional usage The remaining 2, selenocysteine and pyrrolysine, are incorporated into proteins by unique synthetic mechanisms.

The term "amino acid side chain" includes those groups found in: (i) naturally occurring amino acids such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine; (ii) minor amino acids such as ornithine and citrulline; and (iii) unnatural amino acids, beta-amino acids, synthetic analogs and derivatives of naturally occurring amino acids.

As used herein, "comprising," "including," "containing," "characterized by," and grammatical equivalents thereof are inclusive or open-ended terms that do not exclude additional, unrecited elements or method steps. "Comprising" is to be interpreted as including the more restrictive terms "consisting of and "consisting essentially of."

As used herein, "consisting of and grammatical equivalents thereof exclude any element, step, or ingredient not specified in the claim.

As used herein, "consisting essentially of and grammatical equivalents thereof limit the scope of a claim to the specified materials or steps and those that do not materially affect the basic and novel characteristic or characteristics of the claimed invention.

When trade names are used herein, it is intended to independently include the trade name product formulation, the generic drug, and the active pharmaceutical ingredient(s) of the trade name product.

In general, unless defined otherwise, technical and scientific terms used herein have the same meaning as commonly understood by one of ordinary skill in the art to which this invention belongs, and are consistent with general textbooks and dictionaries.

Preferred is a compound of the present invention, wherein n represents an integer of 1 to 6, more preferably an integer of 1-4.

Also preferred is a compound of the present invention, wherein p is an integer of 2 to 6, and more preferably an integer of 2 to 4.

Further preferred is a compound of the invention, wherein the capping group Y is methoxy, ethoxy, propoxy, butoxy, halogen atom, tosylate, isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or 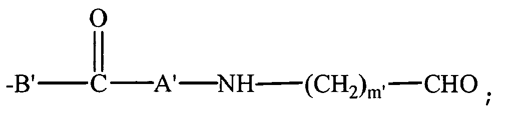 A', B' and m' being defined as A, B and m in relation to the above general formula (I).

In the compound of the present invention, the Pol is preferably selected from poly(ethylene glycol), poly(N-vinylpyrrolidone), N-hydroxy-ethyl methacrylamide copolymer, poly(2-ethyl-2-oxazoline), poly(N-acryloylmorpholine), polyglutamic acid, hyaluronic acid, or polysyalic acid.

Particularly preferred is a compound of the invention, wherein Y is methoxy or 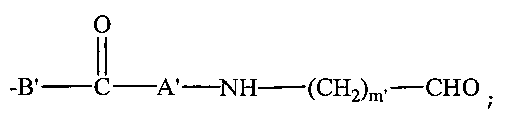 A', B' and m' being defined as A, B and m in relation to the above general formula (I).

Further preferred is a compound of the invention, wherein m is 2 or 3.

The Pol in a compound of the inventionis can be a linear or branched poly(ethylene glycol) (PEG) having a molecular weight (MW) of from 400 to 50000 Da.

Especially preferred is a compound of the invention, wherein PEG is a linear PEG having a molecular weight of about 20000 Da to about 30000 Da.

Also preferred is a compound of the invention, wherein B is O.

Preferably, in the compound of the invention A is -NH- (CH₂)ₚ-CO-; and p is an integer of 2 to 6, more preferably an integer of 2 and 4.

Further preferred is a compound of the invention, wherein Y is methoxy; Pol is linear PEG of about 30000 Da; B is O; p is 2; and m is 3.

In the compound of the present invention, X can represent a cytotoxic drug, preferably a cytotoxic drug selected from natural or synthetic tubulysins; natural or synthetic epothilones; duocarmycines; auristatins; maytansinoids; calicheamycin; mesothelins; and anthracyclins, while n represents an integer of 1 to 4.

In the compound of the present invention L can represent a linker selected from a natural amino acid; β-glutamic acid; aminoadipic acid; Gly-Phe-Leu-Gly; Gly-Leu-Phe-Gly; -Z- (CH₂)ₕ-Z-; -Z- (CH₂)ₕ₁-U-(CH₂)ₕ₂-Z-, U is -COO- or -S-S-; -Z- (CH₂)ₕ-CO-NH-N=; or 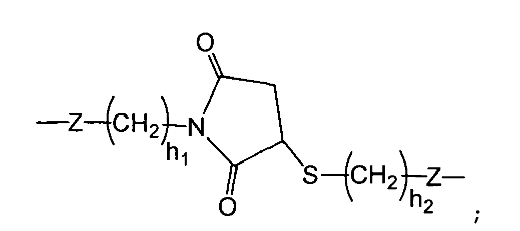 h, h₁ and h₂ are each and independently an integer of 1 to 12; Z represents a single covalent bond, NH or CO; and q is 1.

Preferably, the linker L in the compound of the present invention is selected from -NH- (CH₂₎₂-SS-(CH₂)₂-NH-; -NH- (CH₂)₂-SS- (CH₂₎₂-CO-; -CO- (CH₂)₂-SS- (CH₂)₂-NH-; -CO- (CH₂)₂-SS- (CH₂)₂-CO-; -CO-(CH₂)₂-CO-NH-N=, or -NH- (CH₂)₂-CO-NH-N=.

The polymer aldehyde derivatives of the present invention discussed above may be used to derivatize a variety of materials or molecules, including biomolecules, using any suitable methods. Broadly speaking, any material or molecule having a reactive amine group accessible to the polymer aldehyde derivative according to the invention can be used. Specifically, the derivatization is by reductive amination and coupling methods have been described and are widely known in the art. For example, methods of coupling PEG aldehydes by reductive amination to a material or molecule containing one or more amine groups are disclosed in U.S. Patent 5,252,714, U.S. Patent 5,990,237, and WO 00/24697.

The invention also provides conjugates of the compounds of the invention with materials or molecules such as biomolecules, including polypeptides, proteins, enzymes, antibodies or antibody fragments, phospholipids, lipids, liposomes, nucleosides and oligonucleotides, drugs, dyes, and the surfaces of solid materials that are compatible with living organisms, tissue, or fluids, which are sometimes referred to as biomaterials.

The polymer aldehyde derivatives of the present invention are N-terminus site-specific for the modification, e.g. pegylation, of materials or molecules. The polymer aldehyde derivatives of the present invention form a conjugate with the N-terminus α-amino group of the material or molecule forming a stable secondary amine linkage between the polymer aldehyde compound of the invention and the material or molecule. This site-specific modification, e.g. pegylation, can result in a conjugate, which will allow the modified molecules, e.g. pegylated biomolecules, to retain much or all of their biological activity. The polymer aldehyde derivatives of the present invention can react with any materials or molecules that contain an α-amino group.

Depending on the polymer aldehyde derivative selected the polymer, e.g. polyethylene glycol, may be covalently bonded to a material or molecule at one end (monofunctional polymer aldehyde derivative) or at both ends (bifunctional polymer aldehyde derivative). In the case that the polymer aldehyde derivative is bifunctional, it can be heterobifunctional having an aldehyde group on one terminus and a different functional moiety on the opposite terminus that can be attached via this second functional moiety to a second material or molecule. Such a heterobifunctional dumbbell compound of the present invention can be used, for example, to carry a protein or other biomolecule by an amine linkage on one end and a second material or molecule by another linkage (e.g. sulfone, ester, carbamate, urea, ether, thioether, or disulfide linkage) on the other end. It should be apparent to the skilled artisan that the dumbbell compounds discussed above can be used to carry a wide variety of materials or molecules and combinations thereof, e.g. two different biomolecules. Broadly speaking, almost any substance having chemically reactive functional groups that can be reacted with the functional groups of the dumbbell compounds to form a covalent bond can be modified.

Also provided in accordance with the present invention is a composition comprising a compound of the present invention and a material or molecule that contains an α-amino group.

As stated above, the compounds of the present invention can be covalently bonded to small molecule drugs and/or other biologically active substances such as biomolecules to form a polymer therapeutic. These polymer therapeutics provide altered (improved) pharmacokinetic properties to the attached therapeutic moieties, including a longer in vivo half-life; reduced immunogenicity; reduced toxicity; protection against proteolysis; improved water solubility; and increased pH and thermal stability; while the general activity of the attached therapeutic moieties is retained. Thus, the invention also provides a medicament obtained from a compound according to any one of claims 1 to 12.

Also within in the scope of the present invention is the use of the polymer aldehyde derivative according to the invention for the preparation or manufacture of a medicament of a medicament, or a pharmaceutical composition, especially, a medicament, formulation or pharmaceutical composition for the treatment of cancer.

The pharmaceutical compositions according to the present invention comprise at least one polymer therapeutic obtained from a compound of the invention and, optionally, one or more carrier substances, excipients and/or adjuvants.

Pharmaceutical compositions may additionally comprise, for example, one or more of water, buffers such as, e.g., neutral buffered saline or phosphate buffered saline, ethanol, mineral oil, vegetable oil, dimethylsulfoxide, carbohydrates such as e.g., glucose, mannose, sucrose or dextrans, mannitol, proteins, adjuvants, polypeptides or amino acids such as glycine, antioxidants, chelating agents such as EDTA or glutathione and/or preservatives.

Pharmaceutical compositions may be formulated for any appropriate route of administration, including, for example, topical such as, e.g., transdermal or ocular, oral, buccal, nasal, vaginal, rectal or parenteral administration. The term parenteral as used herein includes subcutaneous, intradermal, intravascular such as, e.g., intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate.

For the preparation of such tablets, pills, semi-solid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

For the treatment of cancer, the dose of the active compound attached to the compound of the invention may vary within wide limits and may be adjusted to individual requirements. Active compounds are generally administered in a therapeutically effective amount. Preferred doses range from about 0.1 mg to about 140 mg per kilogram of body weight per day, about 0.5 mg to about 7 g per patient per day. The daily dose may be administered as a single dose or in a plurality of doses. The amount of active compound that may be combined with the carrier materials to produce a single dosage form will vary depending upon the host treated and the particular mode of administration. Dosage unit forms will generally contain between from about 1 mg to about 500 mg of an active compound.

It will be understood, however, that the specific dose level for any particular patient will depend upon a variety of factors including the activity of the specific compound employed, the age, body weight, general health, sex, diet, time of administration, route of administration, and rate of excretion, drug combination, i.e. other drugs being used to treat the patient, and the severity of the particular disease undergoing therapy.

The present invention also relates to the use of these polymer aldehyde derivatives as a research tool.

Polymer aldehyde derivatives of the present invention can be easily produced by some of the well-known methods in the art.

It is to be noted that the present invention also encompasses all possible combinations of all preferred embodiments.

### Brief description of the figures

Fig. 1: Figure 1 shows the hydrolysis of mPEG-OCONH-βAla-NHS. The averaged T_{1/2} of hydrolysis is 8.48 min for mPEG-OCONH-βAla-NHS.
Fig. 2: Figure 2 shows the hydrolysis of mPEG-OCONH-Gly-NHS. The averaged T_{1/2} of hydrolysis is 1.12 for mPEG-OCONH-Gly-NHS.

### Examples

### Example 1: Preparation of mPEG-OCONH-Gly-CONH-CH₂CH₂CH₂CHO Activation of mPEG30k-OH:

mPEG30k-OH (MW 32314 Da, 5 g, 0.155 mmol) was dried by distillation with 30 ml toluene and then slowly cooled to room temperature. To the solution diluted with 15 ml of CH₂Cl₂, 4-nitrophenyl chloroformate (151.2 mg; 0.774 mmol) and Et₃N (129.4 µl; 0.928 mmol) were added. After 6 hours the reaction mixture was filtered through a Gooch filter G4 and dropped into 250 ml of diethyl ether under stirring. The precipitate, mPEG-OCO-4-nitrophenyl, was recovered by filtration on a Gooch filter G4 and dried under vacuum. The PEG activation degree was spectrophotometrically determined at 400nm after product hydrolysis with NaOH 0.2N releasing 4-nitrophenol (ε = 17000 L·cm·g⁻¹) : 97.5%.

### Synthesis of mPEG-OCONH-Gly-COOH:

Gly (18.48 mg; 0.246 mmol) was dissolved in 10 ml of water/MeCN (3:2). The solution was alkalinised by Et₃N (34.28 *µ*l; 0.246 mmol) and mPEG-OCO-4-nitrophenyl (0.4 g; 0.0123 mmol) was added under stirring. The reaction was let to proceed for 3 hours, then MeCN was removed by evaporation in rotavapor and the solution was acidified to pH 3 by HCl 0.1N. The product mPEG-OCONH-Gly-COOH was extracted by CH₂Cl₂ (4 x 60 ml). The organic phase, dried over anhydrous Na₂SO₄, was concentrated to small volume and following dropped into 100 ml of diethyl ether under stirring. The precipitate, containing mPEG-OCONH-Gly-COOH, was recovered by filtration on a Gooch filter G4 and dried under vacuum.

### Activation of mPEG-OCONH-Gly-COOH

mPEG-OCONH-Gly-COOH (350 mg; 0.011 mmol) was dissolved in 5 ml of CH₂Cl₂ and DCC (6.63 mg; 0.033 mmol) and NHS (2.08 mg; 0.0165 mmol) were added. After 3 h the solution was filtered through a Gooch filter G4 and dropped into 100 ml of diethyl ether under stirring. The product mPEG-OCONH-Gly-NHS was recovered by filtration on a Gooch filter G4 and dried under vacuum. The degree of activation, calculated on the basis of derivative reactivity towards GlyGly as previously reported [Pasut et al (2005) J. Bioact. Compat. Polym. 20:213-215], was 75%.

*Synthesis* of *mPEG-OCONH-Gl y-CONH-CH₂ CH₂CH₂CH(OCH₂CH₃)* ₂ mPEG-OCONH-Gly-NHS (300 mg; 0.009 mmol) was added to a 10 ml solution of 4-amino-butyraldehyde diacetal (4.35 mg; 0.027 mmol) in DMF/CH₂Cl₂ (3:1). The reaction was let to proceed for 3 hours, then the solution was dropped into 100 ml of diethyl ether under stirring. The precipitate, containing mPEG-OCONH-Gly-CONH-CH₂CH₂CH₂CH (OCH₂CH₃)₂, was recovered by filtration on a Gooch filter G4 and dried under vacuum. The final product was characterized by ¹H-NMR.

The derivative is maintained in the diacetal form until before the use in a PEGylation reaction which will be preceded by aldehyde deblocking: the diacetal removal will be achieved through incubation of the PEGylation reagent in phosphoric acid 25 mM pH 2.1 at 50°C for 1 h. The pH will then be adjusted to the required reaction condition for the coupling step with the protein/drug.

### Example 2: Preparation of mPEG-OCONH-βAla-CONH-CH₂CH₂CH₂CHO

The preparation of this derivative has been accomplished using the same chemical strategy reported above for mPEG-OCONH-Gly-CONH-CH₂CH₂CH₂CHO and βAla instead of Gly.

### Example 3: Reactivity comparison of mPEG-OCONH-Gly-NHS and mPEG-OCONH-βAla-NHS

The reactivity of the NHS activated carboxylic group of the two derivatives was compared by evaluating the hydrolysis half-life of the NHS esters in 0.1M borate buffer pH 8. This study can be used to extrapolate the stability of the amide bond of mPEG-OCONH-βAla-CONH-CH₂CH₂CH₂CHO and mPEG-OCONH-Gly-CONH-CH₂CH₂CH₂CHO.

Two experiments for each derivative have been performed using two different concentrations of PEG derivatives in borate buffer, namely 15 mg/ml and 7.5 mg/ml.

One of the derivatives for each experiment is solubilized in 1,4-dioxane and added to a solution of 0.1M borate buffer pH 8 just before recording the UV absorbance at 260 nm. The increase in absorbance was recorded until reaching a stable value. From the graph of the absorbance values against the time course the half-life of hydrolysis can be evaluated.

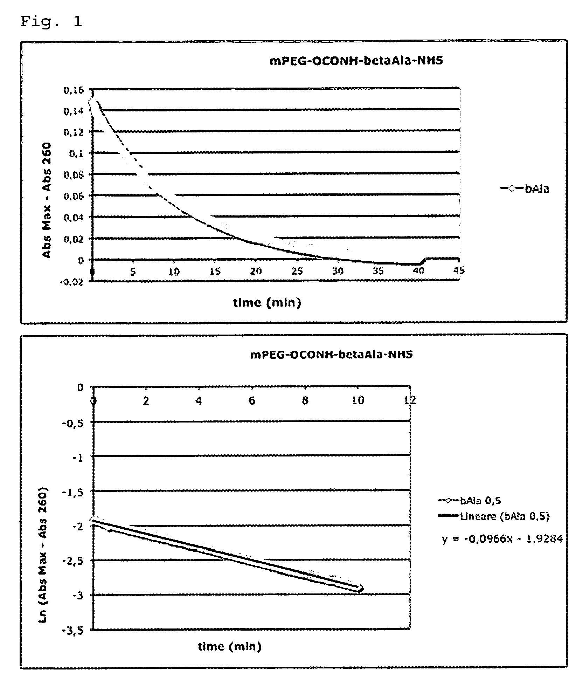

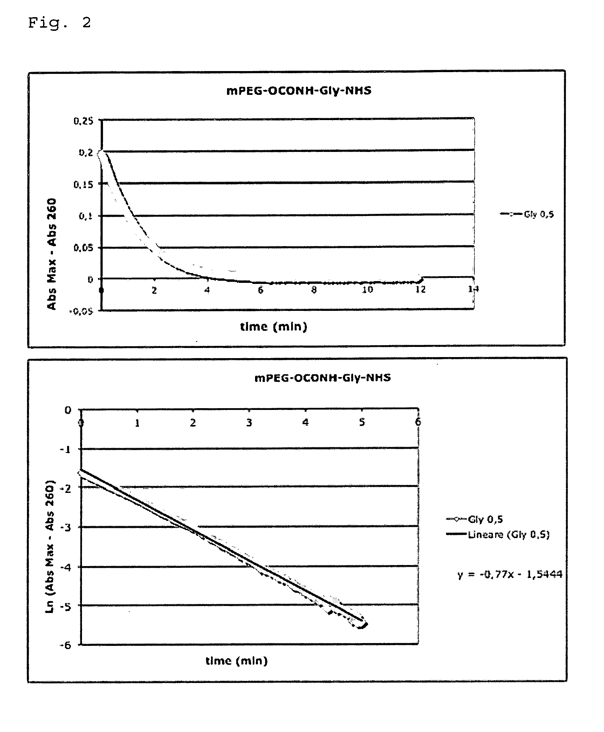

The results are shown in Figure 1 and 2. The averaged T_{1/2} of hydrolysis is 1.12 and 8.48 min for mPEG-OCONH-Gly-NHS and mPEG-OCONH-βAla-NHS, respectively. Accordingly, the derivative including βAla is more stable than the Gly-containing derivative.

### Example 4: Preparation of SPDP-NH-Lys(εNHCO-O-mPEG) CONH-CH₂CH₂CH₂CHO

### Step 1. Synthesis of SPDP-NH-Lys(εNHBOC) -COOH:

NH₂-Lys(εNHBOC) -COOH (263.3 mg; 0.96 mmol) was dissolved in 10 ml of water/MeCN (1:1). The solution was alkalinised by NaOH 0.1N to pH 8 and N-Succinimidyl 3-[2-piridyldithio]-propionate (SPDP) solubilized in 5 ml of DMSO was added (370 mg; 1.18 mmol) under stirring. The reaction was let to proceed for 3 hours, then MeCN was removed by evaporation in rotavapor and the solution was neutralized with HCl 0.1N. The product SPDP-NH-Lys(εNHBOC) -COOH was purified by RP-HPLC using a C18 column eluted with water containing 0.01% TFA (eluent A) and MeCN containing 0.01% TFA (eluent B). Elution gradient: from 10%B to 30%B in 10', at 25' 30%B, at 35' 40%B, at 38' 90%B.

The fractions containing the intermediate were pooled and the solution was evaporated in a rotavapor, allowing the recovery of the product.

### Step 2. Synthesis of SPDP-NH-Lys(εNHBOC) -CONH-CH₂CH (OH) CH₂ (OH) :

SPDP-NH-Lys(sNHBOC)-COOH (180 mg; 0.45 mmol) was dissolved in 5 ml of DMSO. HOBT (164.4 mg; 1.21 mmol) and EDC (233.4 mg; 1.21 mmol) were added under stirring. After 1 h, 3-amino-1,2-propandiol (63 µl; 0.81 mmol).The solution was alkalinised by ET₃N (112.9 µl; 0.81 mmol) under stirring. After 48 h, the product was purified by RP-HPLC using the same eluents of step 1 and a gradient from 10%B to 90%B in 30 min.

The fractions containing the intermediate were pooled and the solution was evaporated in a rotavapor, allowing the recovery of the product.

### Step 3. Synthesis of SPDP-NH-Lys (εNHCO-O-mPEG) CONH-CH₂CH₂CHO

SPDP-NH-Lys (εNHBOC) -CONH-CH₂CH (OH) CH₂ (OH) (120 mg; 0.25 mmol) was dissolved in 4 ml of TFA : CH₂Cl₂ (1:1). After 3 h, the solution was dried in rotavapor to get a dried solid and then dissolved in water: MeCN (1:1). The pH of the solution was brought to 8 by NaOH 0.1N and then mPEG-OCO-4-nitrophenyl (3.25 g; 0.1 mmol), prepared as reported in example 1, was added under stirring. The reaction was let to proceed for 3 hours. Then MeCN was removed by evaporation in rotavapor and the solution was acidified to pH 3 by HCl 0.1N. The product of SPDP-NH-Lys (εNHCO-O-mPEG) CONH-CH₂CH (OH) CH₂ (OH) was extracted by CH₂Cl₂ (4 x 60 ml). The organic phase, dried over anhydrous Na₂SO₄, was concentrated to small volume and following dropped into 400 ml of diethyl ether under stirring. The precipitate, containing SPDP-NH-Lys (εNHCO-O-mPEG) CONH-CH₂CH (OH) CH₂ (OH) , was recovered by filtration on a Gooch filter G4 and dried under vacuum.

SPDP-NH-Lys (εNHCO-O-mPEG) CONH-CH₂CH (OH) CH₂ (OH) (3 g; 0.091 mmol) was dissolved in water (30 mL) and treated for 1 h with sodium periodate (18.9 mg, 0.091 mmol) at 4°C, in the dark. The reaction mixture was dialyzed against distilled water. The aldehyde content of the product was determined with 2,4-dinitrophenylhydrazine (Fields, R., and Dixon, H. B. F. (1971) Micro method for determination of reactive carbonyl groups in proteins and peptides, using 2,4-dinitrophenylhydrazine. Biochem. J. 121, 587-589.). The aqueous solution of the product was stored at 4°C and used as such for conjugation with the given molecule/biologic.

## Claims

1. A compound of the general formula (I): 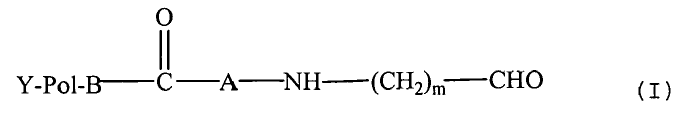 wherein:
Y is a capping group;
Pol is a water-soluble polymer of synthetic or natural origin;
B is N or O;
m is an integer of 1 to 4;
A is a spacer selected from:
-NH- (CH₂)ₚ-CO- ; 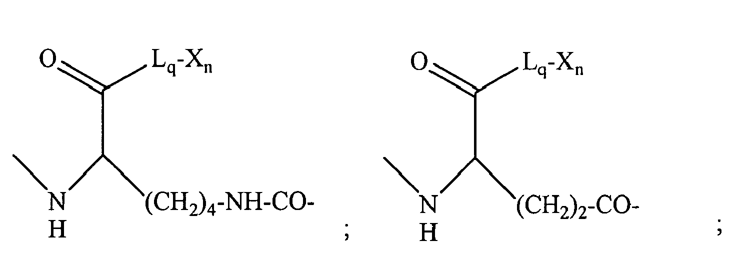 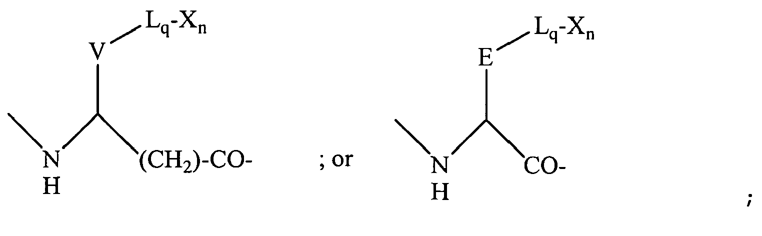
E is the side chain of lysine, cysteine, homocysteine, serine, threonine, ornithine, 2,4 diaminobutyric acid, glutamic acid, aspartic acid, or adipic acid;
V is the side chain of beta-lysine, beta-homolysine or beta-glutamic acid;
L, if present, represents a linker;
X, if present, represents a small molecule drug having a molecular weight of 300 to 1000 Da, a targeting residue, a protecting group, an activated group, or a labeling group, which is bound covalently, or via the respective linker L, to the carbonyl moiety (CO), or the side chain functional group (SH, COOH, NH₂) of E or V, wherein the covalent bond is formed via an ester, amide, carbamate, urea, ether, thioether, sulfur or disulfide bond;
n represents an integer of 0 to 10;
p is an integer of 2 to 10;
q represents an integer of 0 or 1.

2. The compound according to claim 1, wherein the capping group Y is methoxy, ethoxy, propoxy, butoxy, halogen atom, tosylate, isocyanate, hydrazine hydrate, maleimide, orthopyridyl disulfide, N-succinimidyloxy, sulfo-N-succinimidyloxy, 1-benzotriazol, 1-imidazolyloxy, p-nitrophenyloxy, or 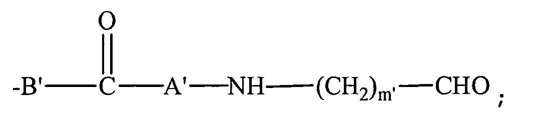 A', B' and m' being defined as A, B and m in claim 1.

3. The compound according to claim 1 or 2, wherein the Pol is selected from poly(ethylene glycol), poly(N-vinylpyrrolidone), N-hydroxy-ethyl methacrylamide copolymer, poly(2-ethyl-2-oxazoline), poly(N-acryloylmorpholine), polyglutamic acid, hyaluronic acid, or polysyalic acid.

4. The compound according to any one of claims 1 to 3, wherein Y is methoxy or 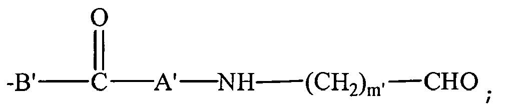 A', B' and m' being defined as A, B and m in claim 1.

5. The compound according to any one of claims 1 to 4, wherein m is 2 or 3.

6. The compound according to any one of claims 1 to 5, wherein the Pol is a linear or branched poly(ethylene glycol) (PEG) having a molecular weight (MW) of from 400 to 50000 Da.

7. The compound according to claim 6, wherein PEG is a linear PEG having a molecular weight of about 20000 Da to about 30000 Da.

8. The compound according to any one of claims 1 to 7, wherein B is O.

9. The compound according to any one of claims 1 to 8, wherein A is -NH-(CH₂)ₚ-CO-; and p is an integer of 2 to 6.

10. The compound according to claim 9, wherein Y is methoxy; Pol is linear PEG of about 30000 Da; B is O; p is 2; and m is 3.

11. The compound according to any one of claims 1 to 8, wherein X represents a cytotoxic drug selected from natural or synthetic tubulysins; natural or synthetic epothilones, duocarmycines, auristatins, maytansinoids, calicheamycin, mesothelins, and anthracyclins; and n is an integer of 1 to 4.

12. The compound according to any one of claims 1 to 8, and 11, wherein L represents a linker selected from a natural amino acid; β-glutamic acid; aminoadipic acid; Gly-Phe-Leu-Gly; Gly-Leu-Phe-Gly; -Z- (CH₂) ₕ-Z-; -Z- (CH₂) ₕ₁-U- (CH₂) ₕ₂-Z-, U is -COO- or -S-S-; -Z- (CH₂) ₕ-CO-NH-N=; or 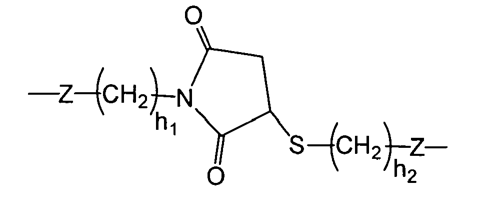 h, h₁ and h₂ are each and independently an integer of 1 to 12; Z represents a single covalent bond, NH or CO;
and q is 1.

13. A composition comprising a compound according to any one of claims 1 to 12, and a material or molecule that contains an α-amino group.

14. The compound according to any one of claims 1 to 12 for use in the preparation of a medicament or a pharmaceutical composition.

15. Use of a compound according to any one of claims 1 to 12 for the preparation of a medicament, the preparation of a pharmaceutical composition, or as a research tool.
